**Europäisches Patentamt**

**European Patent Office**

**Office européen des brevets**

(19)

(11) Veröffentlichungsnummer : **0 057 844**
**B1**

(12) # EUROPÄISCHE PATENTSCHRIFT

(45) Veröffentlichungstag der Patentschrift :
**28.12.83**

(21) Anmeldenummer : **82100483.5**

(22) Anmeldetag : **25.01.82**

(51) Int. Cl.³ : **C 07 C 63/70**, C 07 C 51/62,
C 07 B 9/00

(54) **Verfahren zur Herstellung von Polychlorbenzoylchloriden.**

(30) Priorität : **07.02.81 DE 3104259**

(43) Veröffentlichungstag der Anmeldung :
**18.08.82 Patentblatt 82/33**

(45) Bekanntmachung des Hinweises auf die Patenterteilung : **28.12.83 Patentblatt 83/52**

(84) Benannte Vertragsstaaten :
**CH DE FR GB LI**

(56) Entgegenhaltungen :
DE-A- 2 012 729
GB-A-    833 218
SU-A-    239 311
SU-A-    255 250
US-A- 4 117 006

(73) Patentinhaber : **BAYER AG**
**Zentralbereich Patente, Marken und Lizenzen**
**D-5090 Leverkusen 1, Bayerwerk (DE)**

(72) Erfinder : **Günther, Andreas, Dr.**
**Belaweg 12**
**D-5000 Koeln 80 (DE)**
Erfinder : **Lenthe, Manfred, Dr.**
**Michaelshöhe 40**
**D-5068 Odenthal (DE)**
Erfinder : **Neeff, Rütger, Dr.**
**Berta-von-Suttner-Strasse 22**
**D-5090 Leverkusen 1 (DE)**
Erfinder : **Dankert, Gerhard, Dr.**
**Arthur-Hantzsch-Strasse 44**
**D-5000 Koeln 80 (DE)**

## Verfahren zur Herstellung von Polychlorbenzoylchloriden

Die vorliegende Erfindung betrifft ein Verfahren zur gezielten Herstellung von 3,4-Dichlor-benzoylchlorid, 2,3,4,5-Tetrachlorbenzoylchlorid und Pentachlorbenzoylchlorid durch Kernchlorierung von Benzoylchloriden.

Die Kernchlorierung flüssiger oder gelöster aromatischer Verbindungen durch Umsetzung mit gasförmigem Chlor oder anderen Chlorierungsmitteln ist eine häufig durchgeführte Reaktion. Problematisch ist dabei jedoch in der Regel die gleichzeitige Bildung sowohl von Stellungsisomeren als auch von verschieden hoch chlorierten Produkten. Die einzelnen Verbindungen lassen sich dann nur mit geringer Ausbeute und hohem Aufwand für die Abtrennung gewinnen. Dieses gilt im besonderen Maße auch für die Chlorierung von Benzoylchloriden. So entstehen beispielsweise bei der Chlorierung von Benzoylchlorid in einem Lösungsmittel bei Gegenwart von $FeCl_3$ und einem schwefelhaltigen Co-Katalysator nur 39-49 % an dem gewünschten m-Chlorbenzoylchlorid (vgl. EP-A 0 001 252).

Weiterhin ist bereits aus der SU-PS 255 250 bekannt, daß die Umsetzung von Benzoylchlorid mit Chlorgas in Anwesenheit von Eisensalzen als Katalysator bei Temperaturen von 30-180 °C das 3,4-Dichlorbenzoylchlorid nur zu einem Viertel, das 3,4,5-Trichlorbenzoylchlorid nur zu einem Fünftel, das 2,4,5-Trichlorbenzoylchlorid nur zu einem Sechstel und das 2,3,4,5-Tetrachlorbenzoylchlorid nur zu einem Drittel des jeweiligen Isomerengemisches ergibt, das seinerseits wieder nur einen Bruchteil der Produktmischung ausmacht. Bekannt sind ferner Verfahren zur gezielten Herstellung anderer chlorierter Benzoylchloride. Beispielsweise wird gemäß US-PS 4 117 006 Benzoylchlorid bei Normaldruck mit Chlorgas und einem Katalysator aus 0,3 % Eisen-(III)-chlorid und 0,04 % Jod zu 3-Chlorbenzoylchlorid umgesetzt. Dabei wird eine Ausbeute bis zu 78 % erreicht. Die Reaktionstemperatur beträgt 25 °C und die Reaktionszeit 35 Stunden.

2,3,5,6-Tetrachlorbenzoylchlorid wird nach der britischen Patentschrift 833 218 (entspricht US-PS 3 014 965) aus Benzoylchlorid bei Normaldruck mit Chlorgas und 0,1 % Eisen-(III)-chlorid als Katalysator hergestellt. Die Ausbeute beträgt bis zu 55 %. Die Reaktionstemperatur wird dabei von 110 auf 195 °C gesteigert.

Alle Verfahren haben den Nachteil geringer Ausbeuten und hohen Aufwandes für die Trennung der anfallenden Isomerengemische.

Pentachlorbenzoylchlorid wird bisher aus Pentachlorbenzoesäure und Phosphorpentachlorid hergestellt. (L.G. Zagorskaya, S.I. Barmistrov, S.A. Yashkova, Zh. Obshch. Khim. 32, S. 2612). Letztere wird auf verschiedene Weise gewonnen (Organic Syntheses, Collective Volume 5, S. 890), am einfachsten durch Umsetzung von Benzoesäure mit Chlorgas, wozu diese jedoch z. B. in der fünffachen Menge Chlorsulfonsäure gelöst werden muß (J. Houben, T. Weyl, Methoden der Organischen Chemie, 4. Aufl., Bd. V/3, S. 700). Hierin liegt ein deutlicher Nachteil dieses Verfahrens.

Es wurde nun gefunden, daß man die oben genannten Polychlorbenzoylchloride in relativ hoher Reinheit herstellen kann, wenn man 4-Chlorbenzoylchlorid mit einem Chlorierungsmittel in Gegenwart eines Katalysators bis zum jeweiligen Chlorierungsgrad umsetzt.

Dem erfindungsgemäßen Verfahren liegt folgende Reaktionsfolge zugrunde :

Aus dem 4-Chlorbenzoylchlorid entsteht zunächst 3,4-Dichlorbenzoylchlorid. Dieses reagiert zu 3,4,5- und geringeren Mengen 2,4,5-Trichlorbenzoylchlorid weiter. Beide Isomeren setzen sich bei weiterem Angebot an Chlorierungsmittel zu 2,3,4,5-Tetrachlorbenzoylchlorid um. Aus diesem entsteht schließlich Pentachlorbenzoylchlorid neben wenig Hexachlorbenzol.

Es ist als ausgesprochen überraschend zu bezeichnen, daß das erfindungsgemäße Verfahren das 3,4-Dichlorbenzoylchlorid und die einzelnen Folgeprodukte mit hoher Selektivität und in hohen Ausbeuten liefert, da nach dem Stand der Technik erwarte werden mußte, daß ein komplexes Isomerengemisch entstehen würde und die Reaktion unter den angewendeten Bedingungen entweder auf der Stufe des Tetrachlorbenzoylchlorids stehen bleiben oder aber vorwiegend zu dem unerwünschten Hexachlorbenzol führen würde.

Als Chlorierungsmittel für das erfindungsgemäße Verfahren wird bevorzugt Chlorgas eingesetzt, das in die Reaktionsmischung eingeleitet wird. Es können aber auch flüssiges Chlor oder Chlor freisetzende Chemikalien verwendet werden, vorzugsweise Sulfurylchlorid oder Schwefeldichlorid. Alle Chlorierungsmittel werden bevorzugt im Überschuß eingesetzt.

Die erfindungsgemäße Reaktion wird in Gegenwart eines Katalysators ausgeführt. Bevorzugt wird Eisen-(III)-chlorid, Aluminiumchlorid oder ein äquimolares Gemisch aus Eisen-(III)-chlorid oder Aluminiumchlorid mit Dischwefeldichlorid benutzt. Die Menge des Metallchlorids beträgt vorzugsweise 0,5 bis 10 Gew.-% des eingesetzten Benzoylchlorids.

Für das erfindungsgemäße Verfahren wird das Ausgangsprodukt bevorzugt in flüssiger, lösungsmittelfreier Form eingesetzt. Es können aber auch gegenüber den an der Reaktion beteiligten Stoffen inerte Verdünnungsmittel verwendet werden, vorzugsweise Tetrachlorkohlenstoff.

Die Temperatur der erfindungsgemäßen Reaktion kann in dem weiten Bereich zwischen dem Erstarrungs- und dem Siedepunkt des Reaktionsgemisches variiert werden, d. h. für die Di- bis Tetrachlorierung zwischen 20 und 200 °C, vorzugsweise zwischen 55 und 120 °C, und für die Pentachlorierung zwischen 80 und 200 °C, vorzugsweise zwischen 100 °C und 140 °C.

Das erfindungsgemäße Verfahren kann bei allen Drücken betrieben werden, bei denen das eingesetzte Chlor noch gasförmig ist, vorzugsweise zwischen 1 und 5 bar. Bei Einsatz flüssiger Chlorierungsmittel kann der Druck noch weiter variiert werden.

Die erfindungsgemäße Reaktion wird bevorzugt diskontinuierlich bezüglich der organischen Produkte in einem Rührkessel oder einer Blasensäule durchgeführt. Gegebenenfalls werden die Verbindungen, die weniger hoch chloriert sind als das gewünschte Produkt, nach ihrer Abtrennung vom Produkt erneut in die Reaktion zurückgeführt. Die Reaktion kann auch kontinuierlich entweder einstufig in einem Rührkessel oder einer Blasensäule oder mehrstufig in einer Rührkesselkaskade oder einer mehrstufigen Blasensäule ausgeführt werden, sowohl in Gleich- als auch in Gegenstromblasensäulen.

Die Reaktionszeit des erfindungsgemäßen Verfahrens steigt mit fallender Katalysatorkonzentration, fallender Temperatur, fallender Ausgangskonzentration an Benzoylchlorid und Chlorierungsmittel, bzw. fallendem Druck bei Verwendung von Chlorgas. Die erreichbare Ausbeute sinkt bei allen Produkten, außer beim Pentachlorbenzoylchlorid, mit steigender Temperatur ab. Die Reaktionszeiten können jedoch bei fast maximaler Ausbeute deutlich verringert werden, indem man die Chlorierung mit hoher Temperatur beginnt, z. B. 120 °C, und mit fortschreitender Umsetzung die Temperatur senkt, z. B. bis auf 60 °C. Es ist möglich, auf eine Aufheizung der Reaktionsmischung vor Beginn der Reaktion zu verzichten, da sich das Gemisch durch die Reaktionswärme schnell selbst von z. B. 20 °C auf die gewünschte Reaktionstemperatur erwärmt.

Bei gleichem Chlorstrom ist in der Blasensäule die Reaktionszeit kürzer als im Rührkessel. Die Reaktion muß bei allen Produkten zu einem bestimmten optimalen Zeitpunkt abgebrochen werden, um eine maximale Ausbeute an dem gewünschten Produkt zu erhalten. Im Falle der Rückführung des Edukts und der Vorprodukte in die Reaktion wird diese schon vor dem Erreichen der maximalen Produktkonzentration beendet, um die Menge an unerwünschtem Folgeprodukt zu verringern. Die Reaktionszeit liegt im allgemeinen zwischen 0,5 und 50 Stunden.

Die Abtrennung und Reindarstellung der Reaktionsprodukte des erfindungsgemäßen Verfahrens geschieht durch Vakuumdestillation über eine gut wirkende Kolonne. Es kann vorteilhaft sein, größere Anteile an Hexachlorbenzol vor der Destillation aus der Reaktionsmischung zu entfernen. Dazu wird diese in geeigneten Lösungsmitteln, z. B. Aceton gelöst, wobei die Hauptmenge des Hexachlorbenzols ungelöst zurückbleibt.

Das erfindungsgemäße Verfahren hat den Vorteil, daß die Bildung mehrerer Isomeren des Di- und des Tetrachlorbenzoylchlorids vermieden wird, so daß die Trennkosten und der Anfall an unerwünschten Nebenprodukten gering sind.

Günstig ist weiterhin, daß es die hohe Ausbeute der Zwischenprodukte 3,4-Dichlorbenzoylchlorid und 2,3,4,5-Tetrachlorbenzoylchlorid erlaubt, diese Stoffe ohne vorzeitigen Abbruch der Chlorierung und anschließende Nachchlorierung der abgetrennten Vorprodukte herzustellen. Ein weiterer Vorteil des erfindungsgemäßen Verfahrens ist es, daß das Pentachlorbenzoylchlorid hierdurch erstmals ohne Verwendung von Lösungsmitteln produziert werden kann. Schließlich ist vorteilhaft, daß die genannten Benzoylchloride in demselben Reaktor und unter ähnlichen Bedigungen hergestellt werden können.

Die erfindungsgemäß herstellbaren Benzoylchloride sind wertvolle Zwischenprodukte zur Herstellung von Farbstoffen, von Insektizidin, von Herbiziden (vgl. G. Pagani, A. Baruffini, M. Mazza, L. Vicarini, G. Caccialauza, Farmaco, Ed. Sci. 28 (1973), S. 570-589) und von Flammschutzmitteln (vgl. Patentschriften US 3 950 307, US 3 953 397, US 3 959 216 und US 3 959 217).

Das erfindungsgemäße Verfahren wird durch die folgenden Beispiele veranschaulicht. Die einzelnen Verbindungen wurden durch GC-MS-Kopplung und durch NMR-Spektren der Destillatfraktionen identifiziert.

Beispiel 1

3,4-Dichlorbenzoylchlorid

5 600 g (32 Mol) 4-Chlorbenzoylchlorid, 28 g (0,17 Mol) Eisen-(III)-chlorid und 23 g (0,17 Mol) Dischwefeldichlorid werden in einer Blasensäule, die mit Stickstoff begast wird, bei 5 bar bis 60 °C aufgeheizt. Die Blasensäule hat einen Durchmesser von 10 cm und eine Höhe von 90 cm und besteht aus Edelstahl. Die Begasung geschieht durch einen Siebboden von 1,5 mm Dicke, der 19 Bohrungen mit 1 mm Durchmesser aufweist. Nachdem von Stickstoff auf Chlorgas im Überschuß umgestellt worden ist, wird die Reaktionsmischung 2 Stunden lang auf 60 °C und 5 bar gehalten. Es werden 94 Molprozent 3,4-Dichlorbenzoylchlorid neben 3 %* Ausgangsprodukt und 3 % Trichlorbenzoylchlorid erhalten. Die Rektifikation bei 15 mbar über eine Kolonne ergibt das Produkt mit praktisch 100 % Reinheit.

* alle Prozentangaben beziehen sich auf Molprozent

Beispiel 2

3,4-Dichlorbenzoylchlorid

44 g (0,25 Mol) 4-Chlorbenzoylchlorid werden in einem Glaskolben unter Rühren bei 60 °C und 1 bar in 10 Minuten mit einer Mischung aus 445 g (3,5 Mol) Sulfurylchlorid, 4,4 g (0,033 Mol) Aluminiumchlorid und 4,5 g (0,033 Mol) Dischwefeldichlorid versetzt und anschließend bei 60 °C nachgerührt. Nach 10 Stunden werden 95 Molprozent 3,4-Dichlorbenzoylchlorid neben 1 % Ausgangsprodukt und 4 % Trichlorbenzoylchlorid erhalten.

Beispiel 3

3,4-Dichlorbenzoylchlorid

350 g (2 Mol) 4-Chlorbenzoylchlorid und 3,5 g (0,02 Mol) Eisen-(III)-chlorid werden in einem Glaskolben unter Rühren bei 55 °C und 1 bar in 1 Stunde mit 825 g (8 Mol) Schwefeldichlorid versetzt und anschließend bei 55 °C nachgerührt. Nach 19 Stunden werden 99 Molprozent 3,4-Dichlorbenzoylchlorid neben 0,5 % Ausgangsprodukt und 0,5 % Trichlorbenzoylchlorid erhalten.

Beispiel 4

3,4-Dichlorbenzoylchlorid

In 175 g (1 Mol) 4-Chlorbenzoylchlorid, 8,75 g (0,05 Mol) Eisen-(III)-chlorid und 700 g Tetrachlorkohlenstoff wird in einem Glaskolben unter Rühren bei 60 °C und 1 bar Chlorgas im Überschuß eingeleitet. Nach 15 Stunden werden 96 Molprozent 3,4-Dichlorbenzoylchlorid neben 0,5 % Ausgangsprodukt und 3,5 % Trichlorbenzoylchlorid erhalten.

Beispiel 5

2,3,4,5-Tetrachlorbenzoylchlorid

Versuchsdurchführung wie Beispiel 1. Nach 38 Stunden werden 91 Molprozent 2,3,4,5-Tetrachlorbenzoylchlorid neben 7,5 % Pentachlorbenzoylchlorid und 1,5 % Hexachlorbenzol erhalten.

Beispiel 6

2,3,4,5-Tetrachlorbenzoylchlorid

In 350 g (2 Mol) 4-Chlorbenzoylchlorid und 3,5 g (0,02 Mol) Eisen-(III)-chlorid wird in einem Glaskolben unter Rühren bei 120 °C und 1 bar Chlorgas im Übershuß eingeleitet. Nach 8 Stunden werden 79 Molprozent Tetrachlorbenzoylchlorid neben 8 % Trichlorbenzoylchlorid, 11 % Pentachlorbenzoylchlorid und 2 % Hexachlorbenzol erhalten.

Beispiel 7

2,3,4,5-Tetrachlorbenzoylchlorid

In 98 kg (560 Mol) 4-Chlorbenzoylchlorid und 0,9 kg (5,6 Mol) Eisen-(III)-chlorid wird in einem Emaille-Rührkessel bei 20 °C Chlorgas im Überschuß eingeleitet, wobei der Druck nach Anstieg auf 5 bar auf diesem Wert gehalten wird. Eine Temperatur von 60 °C ist nach 1 Stunde erreicht und wird anschließend durch Kühlen, später durch Heizen beibehalten. Nach 45 Stunden werden 90 Molprozent 2,3,4,5-Tetrachlorbenzoylchlorid neben 8,5 % Pentachlorbenzoylchlorid und 1,5 % Hexachlorbenzol erhalten. Die Rektifikation bei 15 mbar über eine Kolonne mit 15 theoretischen Böden ergibt das Produkt mit 99,9 % Reinheit.

Beispiel 8

Pentachlorbenzoylchlorid

Versuchsdurchführung wie Beispiel 1, jedoch 56 g (0,35 Mol) Eisen-(III)-chlorid, kein Dischwefeldichlorid und 120 °C als Reaktionstemperatur. Nach 9 Stunden werden 79 Molprozent Pentachlorbenzoylchlorid neben 3 % 2,3,4,5-Tetrachlorbenzoylchlorid und 18 % Hexachlorbenzol erhalten.

Das Produktgemisch wird abgekühlt und unter Rückfluß mit der dreifachen Gewichtsmenge Aceton versetzt. Bei Raumtemperatur wird vom Ungelösten abfiltriert und die Lösung eingedampft. Der Eindampfrückstand besteht aus 93 Molprozent Pentachlorbenzoylchlorid, 3,5 % Tetrachlorbenzoylchlorid und 3,5 % Hexachlorbenzol. Der Filterkuchen ist fast völlig reines Hexachlorbenzol. Durch Vakuumrektifikation wird das Pentachlorbenzoylchlorid rein erhalten.

**Ansprüche**

1. Verfahren zur Herstellung von 3,4-Dichlorbenzoylchlorid, 2,3,4,5-Tetrachlorbenzoylchlorid oder Pentachlorbenzoylchlorid, dadurch gekennzeichnet, daß man 4-Chlorbenzoylchlorid mit einem Chlorierungsmittel in Gegenwart eines Katalysators bis zum jeweiligen Chlorierungsgrad umsetzt.

2. Verfahren gemäß Anspruch 1, dadurch gekennzeichnet, daß man als Katalysator Eisen-(III)-chlorid oder Aluminiumchlorid in einer Menge von 0,5 bis 10 Gew.-% des eingesetzten 4-Chlorbenzoylchlorids anwendet.

3. Verfahren gemäß Ansprüchen 1 und 2, dadurch gekennzeichnet, daß man auf 1 Mol des als Katalysator eingesetzten Metallchlorids 0,5 bis 10 Mol Dischwefeldichlorid zusetzt.

4. Verfahren gemäß Anspruch 1, dadurch gekennzeichnet, daß man die Umsetzung in einem indifferenten Lösungsmittel durchführt.

5. Verfahren gemäß Anspruch 4, dadurch gekennzeichnet, daß man als Lösungsmittel CCl₄ verwendet.

6. Verfahren gemäß Anspruch 1, dadurch gekennzeichnet, daß man als Chlorierungsmittel Sulfurylchlorid oder Schwefeldichlorid einsetzt.

7. Verfahren gemäß Anspruch 1, dadurch gekennzeichnet, daß man das Chlorierungsmittel im Überschuß einsetzt.

8. Verfahren gemäß Anspruch 1, dadurch gekennzeichnet, daß man die Reaktion bei Temperaturen zwischen 20 und 200 °C, vorzugsweise zwischen 55 und 120 °C, durchführt.

9. Verfahren gemäß Anspruch 1, dadurch gekennzeichnet, daß man die Reaktion bei einem Druck zwischen 1 und 5 bar durchführt.

10. Verfahren gemäß Anspruch 1, dadurch gekennzeichnet, daß man die Benzoylchloride, die weniger als die gewünschte Anzahl an Chlorsubstituenten enthalten, aus der Reaktionsmischung abdestilliert und erneut den Reaktionsbedingungen unterwirft.

**Claims**

1. Process for the preparation of 3,4-dichlorobenzoyl chloride, 2,3,4,5-tetrachlorobenzoyl chloride or pentachlorobenzoyl chloride, characterised in that 4-chlorobenzoyl chloride is reacted with a chlorinating agent in the presence of a catalyst, until the particular degree of chlorination is achieved.

2. Process according to Claim 1, characterised in that iron(III) chloride or aluminium chloride, in a quantity of from 0.5 to 10 % by weight of the 4-chlorobenzoyl chloride employed, is used as the catalyst.

3. Process according to Claims 1 and 2, characterised in that 0.5 to 10 mols of disulphur dichloride are added per mol of the metal chloride employed as the catalyst.

4. Process according to Claim 1, characterised in that the reaction is carried out in an inert solvent.

5. Process according to Claim 4, characterised in that $CCl_4$ is used as the solvent.

6. Process according to Claim 1, characterised in that sulphuryl chloride or sulphur dichloride is employed as the chlorinating agent.

7. Process according to Claim 1, characterised in that the chlorinating agent is employed in excess.

8. Process according to Claim 1, characterized in that the reaction is carried out at temperatures between 20 and 200 °C, preferably between 55 and 120 °C.

9. Process according to Claim 1, characterised in that the reaction is carried out under a pressure of between 1 and 5 bars.

10. Process according to Claim 1, characterised in that the benzoyl chlorides which contain less than the desired number of chlorine substituents are distilled off from the reaction mixture and again subjected to the reaction conditions.

**Revendications**

1. Procédé de production de chlorure de 3,4-dichlorobenzoyle, de chlorure de 2,3,4,5-tétrachlorobenzoyle ou de chlorure de pentachlorobenzoyle, caractérisé en ce qu'on fait réagir du chlorure de 4-chlorobenzoyle avec un agent de chloration en présence d'un catalyseur jusqu'au degré de chloration correspondant.

2. Procédé suivant la revendication 1, caractérisé en ce qu'on utilise comme catalyseur le chlorure de fer-(III) ou le chlorure d'aluminium en une quantité de 0,5 à 10 % en poids du chlorure de 4-chlorobenzoyle utilisé.

3. Procédé suivant les revendications 1 et 2, caractérisé en ce qu'on ajoute 0,5 à 10 moles de chlorure de soufre par mole du chlorure métallique utilisé comme catalyseur.

4. Procédé suivant la revendication 1, caractérisé en ce qu'on conduit la réaction dans un solvant indifférent.

5. Procédé suivant la revendication 4, caractérisé en ce qu'on utilise $CCl_4$ comme solvant.

6. Procédé suivant la revendication 1, caractérisé en ce qu'on utilise le chlorure de sulfuryle ou le dichlorure de soufre comme agent de chloration.

7. Procédé suivant la revendication 1, caractérisé en ce qu'on utilise l'agent de chloration en excès.

8. Procédé suivant la revendication 1, caractérisé en ce qu'on conduit la réaction à des températures comprises entre 20 et 200 °C, de préférence entre 55 et 120 °C.

9. Procédé suivant la revendication 1, caractérisé en ce qu'on conduit la réaction à une pression comprise entre 1 et 5 bars.

10. Procédé suivant la revendication 1, caractérisé en ce que les chlorures de benzoyle qui contiennent des substituants chloro en nombre inférieur au nombre désiré sont chassés par distillation du mélange réactionnel et soumis de nouveau aux conditions réactionnelles.